# EUROPEAN PATENT APPLICATION

(11) **EP 4 406 519 A1**
(43) Date of publication of application: **31.07.2024**
(21) Application number: 22814128.9
(22) Date of filing: 22.09.2022
(51) Int. Cl.: A61F 5/34, A47C 7/00, A47C 7/02, A47C 7/14, A47C 7/42, A47C 7/46

(54) **LUMBAR LORDOSIS FACILITATOR DEVICE**

(30) Priority: 24.09.2021 ES 202100365 U
(71) Applicant: SERRANO BELLES, Felix, 12003 Castello de la Plana (ES); Serrano Belles, Francisco Javier, 12004 Castelló de la Plana Castellón (ES)
(72) Inventor: SERRANO BELLES, Felix, 12003 Castello de la Plana (ES); Serrano Belles, Francisco Javier, 12004 Castelló de la Plana Castellón (ES)
(74) Representative: Espiell Gomez, Ignacio
(86) International application number: PCT/ES2022/000037
(87) International publication number: WO 2023/047001

(57) **Abstract**

Lumbar lordosis facilitator device composed of one or several fluid bags (1). Each bag (1) has one or several fluid entry points (2) connected to a pressurized fluid source (7) so that they allow the filling of the bag or bags (1) from a primary end to one or several fluid exit points (3) located in a way that allow the evacuation of the fluid to a secondary end on the opposite side of the primary end. The device also has a fluid inner pressure regulating system that can employ several sensor systems (8,9,10).

## Description

### FIELD OF TECHNIQUE NUMBER

The following invention refers to a lumbar lordosis facilitator device which can be adapted to all sorts of seats, and which encompasses elements to ensure the correct position of the user's back thus reducing the risk of injuries.

### TECHNIQUE STATUS

It is common for people who spend too much time seated to have back problems due to a bad posture. The solution is usually to modify the back of the chair so that the user's back may adopt a more appropriate position. However, this does not guarantee that the user leans back on the chair properly on most occasions.

Within the technique, there is a system with a fluid bag that can be placed on the chair. This system is partially filled with fluid. Hence, when the user sits on the front of the bag, the fluid is transferred to the back and creates a thrust that pushes the user into the right position. This system is fairly efficient and is considered the closest one to this invention. However, this system largely relies on the user's position. It does not work if the user sits excessively forward or backward and may even have counterproductive effects.

The applicant does not know of any solution that is as efficient as the one that is being claimed.

### BRIEF EXPLANATION OF THE INVENTION

This invention consists of a lumbar lordosis facilitator device according to the claims. Its configurations solve the reviewed problems and provide notable advantages which are evident to experts on the matter.

This invention is a lumbar lordosis facilitator device composed of one or more fluid bags as the one described in the technique status. However, in this case, each bag has one or several entries of gas or another fluid which are hooked to a source of pressurized fluid - generally air - located in a way that allows the filling of the bags from a primary end which will be placed at the top of the chair (near the back) while in service. It also has one or several fluid exit points located in a way that allow the evacuation of the fluid, at least through the opposite end or secondary end. The fluid source could be a compressor, a gas cartridge, a pressure pump... although it is not particularly relevant which specific type it is. Furthermore, this system includes a regulating system for the fluid's inner pressure. This system should preferably be a smart system, although it may be formed of differential valves at the entry point (between the source and the bag) and/or at the exit point.

In the preferred solution, the pressure regulating system includes one or several primary sensors (primary sensor system) near the entry point which are located at the top of the chair (near the back) while in service, and one or several secondary sensors (secondary sensor system) for the remaining range of the bag or bags. These two sensor systems detect or confirm the presence of a user. It also has one or several tertiary sensors (tertiary sensor system) at the front of the bag or bags in order to detect the curving of the secondary end of the bag. The sensor systems may include different types as will be later indicated.

In one of its configurations, the device is formed of two or more bags connected to each other or separated though facing the same direction so that, in the latter case, each of the user's gluteus has a different pressure thus controlling comfort. These bags are preferably joint by a structure and/or inside a case or cases.

Additional exit points may be placed at other locations apart from the secondary end. For example, to compensate when the user sits back once the path to the exit point is closed. In other words, when the available volume of the bag is reduced, pressure is brought down through these additional exit points.

In another configuration, the exit point is open, without valves that may close the path, and the entry point is set to be hooked or to insert fluid from the source when the user's presence is detected. This insertion may be based on the detected weight and position of the user (e.g. using data tables relating the weight to the pressure to be used).

Other variants will be described below which are defined in dependent claims.

### DESCRIPTION OF THE DRAWINGS

In order to understand this invention better, the following figures have been included:
Figure 1. Longitudinal section of a bag according to a configuration example.
Figure 2. Longitudinal section of a second example in service.
Figure 3 (A-E). Five different configuration examples of the device in a schematic top view.

### CONFIGURATION MODES OF THE INVENTION

We will provide a brief description of a configuration mode of the invention as an illustrative though not limited example of it.

The configuration of the device shown in the figures comes from one or several inflatable bags (1), each of which has one or several entry points (2) and one or several exit points (3) of air or another fluid, in this case represented in two opposite ends. The entry point (2) is represented over a primary end, the first to be filled, and the exit point (3) over a secondary end. This secondary end will be emptied of fluid while in service. When two or more bags (1) are placed, it is preferable that these are joint by a structure (4) made of fabric, nonwoven material... forming a cushion that is placed on the chair or embedded in it (3D figure). The bags (1) cover all the area where the chair's user may lean back so that the support point is irrelevant.

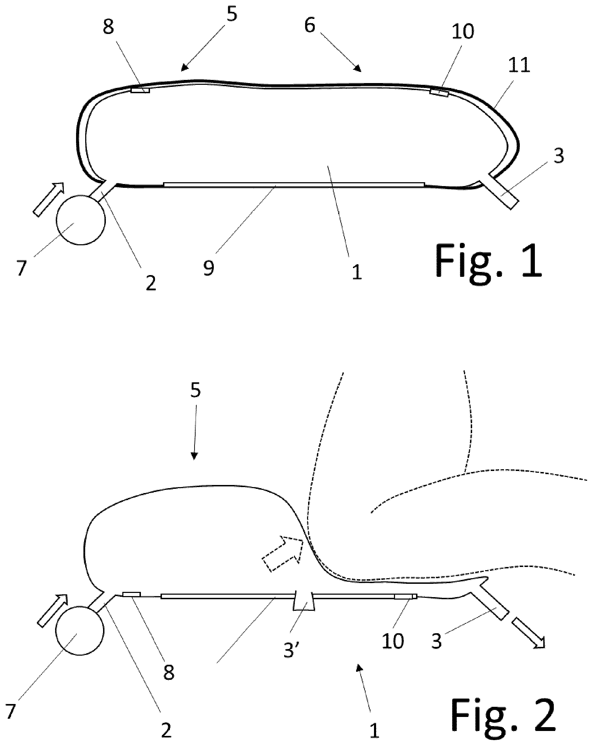

This invention will be described based on the configuration of figures 1 and 2. Two segments (5,6) are defined in each bag (1), in this case with an entry point (2) and an exit point (3), and a regulating system of the fluid's inner pressure. The first segment (5) is attached to the entry point (2) and the second segment (6) is attached to the exit point (3). Both the exit point (3) and the entry point (2) usually have valves to close or open the path of the fluid automatically or by means of an electronic controller. They may have check valves. An applicable example is a duckbill valve or a spring that opens when the pressure difference at both sides goes over a prefixed value. The exit point (3) may not have a valve if the inner surface of the bag (1) closes the path sufficiently when both sides approach under the weight of the user.

The entry point (2) is connected to a source (7) of gas or another pressurized fluid, such as a compressor, a compressed gas cartridge, an accumulator connected to a compressor, a pressure pump... or any such element. A manometer or suchlike device monitors the proper pressure of the fluid inside the bag (1). The exit point (3) may be open to the exterior or to a fluid deposit from where the source (7) takes it back, forming a closed circuit. An additional exit point (3') may be included near the entry point (2) with a differential or electronic valve to release the fluid if a pressure range is exceeded inside. If the entry point (2) has a solenoid valve, it may also work as an additional exit point (3') opening and closing without activating the source (7). This additional exit point (3') may be placed at any point of the bag (1), such as the middle area.

Each bag (1) may have a case (11) which has several functions. Firstly, this case (11) defines an inflation top limit of the bag (1) so that inner pressure may be increased without affecting the volume. Furthermore, it allows the modification of the aesthetic aspect, touch, or offers finishes against stains and suchlike advantages.

Each bag (1) has a primary presence sensor system (8) in the first segment (5), a secondary presence sensor system (9) in the bag (1), and a tertiary sensor system (10) to detect the curving of the bag (1) which is located at the front of the bag (1). Each of these sensor systems (8,9,10) may be repeated to ensure the detection or to take measurements at several points.

The primary sensor system (8) and the secondary second sensor system (9) are sensors that detect the presence of the user. They will usually detect weight, although they may be buttons set on springs, pressure sensors, conductivity sensors... The tertiary sensor (10) detects the curving of the bag (1) at the front of the second segment (6) and it may detect the deformation of the bag (1), inner pressure of the bag (1), fluid path through the exit point (3), etc. Having two sensor systems (8,9) allows to know the position of the user - whether more toward the front or toward the back - on the device. It is possible to place three or more sensor systems (8,9) in order to achieve higher precision in the detection of presence and position.

If the sensor systems (8,9) detect the presence of the user by weight, it is possible to regulate the pressure according to tables that indicate which pressure must be supply to the source (7) according to the user's weight. The pressure may be measured directly or based on the pump's power.

The sensor systems (8,9) must detect the user's presence in either a direct way, such as through weight, or indirectly. An example of an indirect way would be by detecting the fluid path through the exit point (3) as a way to check that someone has sat at the secondary end.

The exit point (3) at the opposite end of the entry point may be open, with no valve, if fluid is introduced once a person who has sat is detected, being the weight of this person the element that closes the fluid path toward the exit point by squeezing the two walls of the bag (1) in a way that arrives at the situation shown in figure 2.

The interior of each bag (1) may have separating elements to ensure that each side of the bag (1) are separated when the person gets up - in other words, when the user's presence is no longer there. For example, it could have teeth or foam rubber cogs or made of some soft material placed over an inner surface maintaining a gap between both sides of the bag (1) once the weight is removed so that the bag goes back to its original position (1).

While in service, the device is placed on a chair or is embedded in it so that the second segment (6) remains at the front of the chair. When the user sits on the chair, couch, seat... placing his/her buttocks over the devices, he/she squashes the bag (1) or bags (1) starting at the second segment (6) toward the back. If he/she only leans on the second segment (6), the second segment is emptied (6) through the exit point (3) while the first segment (5) swells until the correct thrust is produced. The curving of the device redirects the user's body setting him/her into the correct lumbar position. If he/she leans on the second segment (6) and partially on the first segment (5), depending on the pressure it has and its difference compared to the desired pressure, fluid will be removed or injected until the correct thrust is produced facilitating once more the lumbar lordosis. However, if the user leans on the second segment, the bag (1) will be fully emptied of fluid as it is understood that the user is properly seated and leaning on the hypothetical back, so that the primary sensor system (8) detects the seated user.

The secondary sensor system (9) detects the user's presence while the other sensors detect whether it is necessary to empty the bag (tertiary sensor system (10) and primary sensor system [8]). The tertiary sensor system (10) may be replaced for a differential pressure valve at the exit point (3), a flow sensor at one or several exit points (3) ... It is also possible - although it is a less preferred option - to place a differential pressure valve at the entry point (2) to complement or replace the primary sensor (8).

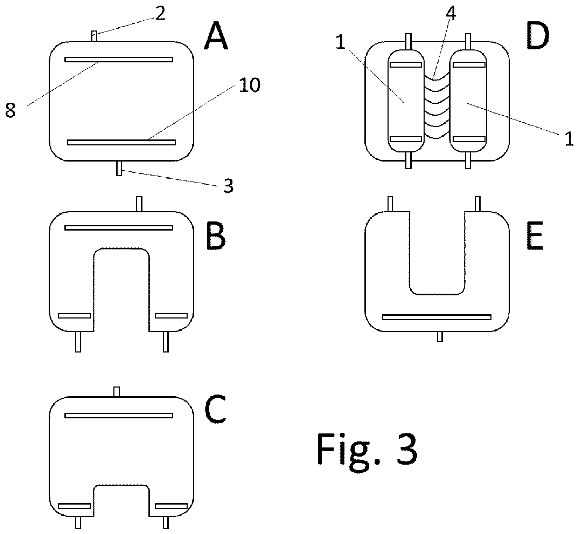

The device has several possible forms which are shown in some examples in figures 3A-3E. The basic idea is that each of the user's gluteus is supported by a bag (1) or bags (1), and this operation may be carried out in many ways.

An electronic switchboard may control all the elements of the device while having an on- and-off switch. The device may be battery powered or by a cord connected to the grid or by a cord connected to a car battery, etc. If wanted, the fluid may be warmed as it is introduced into the bag (1) by a resistor placed at the entry point (2).

## Claims

1. Lumbar lordosis facilitator device composed of one or several fluid bags (1) having each bag (1) the characteristic of having one or several fluid entry points (2) connected to a pressurized fluid source (7) located so that they allow the filling of the bag or bags (1) from a primary end to one or several fluid exit points (3) so that they allow the evacuation of fluid from a secondary end on the opposite side of the primary one. It also features a fluid inner pressure regulating system.

2. Lumbar lordosis facilitator device which, according to claim 1, **characterized by** a pressure regulating system with at least a differential pressure valve.

3. Lumbar lordosis facilitator device, according to claim 1, **characterized by** a pressure regulating system that has:
One or several primary sensors which form a primary sensor system (8) near the entry point (2) and one or several sensors forming a secondary sensor system (9) to detect the presence of a user; and one or several tertiary sensors forming a tertiary sensor system (10) to detect the curving of the bag (1) or the fluid path to the secondary end of the bag (1).

4. Lumbar lordosis facilitator device, according to claim 1, **characterized by** being configured by two or more independent bags (1) facing the same direction.

5. Lumbar lordosis facilitator device, according to claim 4, **characterized by** having the bags (1) joint by a structure (4).

6. Lumbar lordosis facilitator device, according to claim 1, **characterized by** having check valves at the entry point (2) and/or the exit point (3).

7. Lumbar lordosis facilitator device, according to 1, **characterized by** having the exit point (3) connected to a fluid deposit which is connected to the source (7).

8. Lumbar lordosis facilitator device, according to claim 1, **characterized by** having each bag (1) containing separating elements that ensure a gap between both sides of the bag (1).

9. Lumbar lordosis facilitator device, according to claim 1, **characterized by** the inner sides of each bag (1) that close the fluid path when they lean against each other.

10. Lumbar lordosis facilitator device, according to claim 1, **characterized by** having additional exit points (3') outside the secondary end.

11. Lumbar lordosis facilitator device, according to claim 1, **characterized by** having an outer case (11) for the bag (1).

12. Lumbar lordosis facilitator device, according to claim 1, **characterized by** having the exit point (3) open and the entry point (2) configured to activate itself when the user's presence is detected.

13. Lumbar lordosis facilitator device, according to claim 12, **characterized by** a source (7) that is activated based on the weight and position of the user which are detected by the sensor systems (8,9).
